# FASCICULE DE BREVET EUROPEEN

(11) **EP 0 978 724 B1**
(45) Date de publication et mention de la délivrance du brevet: **02.10.2002**
(21) Numéro de dépôt: 99401781.2
(22) Date de dépôt: 15.07.1999
(51) Int. Cl.: G01N 33/50, G01N 33/72

(54) **Réactif pour la mesure de l'hémoglobine et la détermination des leucocytes dans un échantillon de sang**
Reagenz zum Assay von Hämoglobin und zur Bestimmung der Leukozyten in einer Blutprobe
Reagent for the assay of hemoglobin and the determination of leukocytes in a blood sample

(30) Priorité: 04.08.1998 FR 9810010
(43) Date de publication de la demande: 09.02.2000
(73) Titulaire: ABX, 34184 Montpellier Cedex 4 (FR)
(72) Inventeur: Veriac, Sylvie, 34000 Montpellier (FR); Champseix, Henri, 34980 Montferrier sur Lez (FR)
(74) Mandataire: Bezault, Jean

(56) Documents cités:
- EP-A- 0 430 750
- EP-A- 0 660 113
- EP-A- 0 743 519
- FR-A- 2 735 578
- US-A- 4 751 179

## Description

L'invention se rapporte aux analyses hématologiques.

Elle concerne plus particulièrement un réactif pour la mesure de l'hémoglobine et la détermination des leucocytes dans un échantillon de sang.

La mesure de la concentration de l'hémoglobine et la détermination des leucocytes, en particulier de certaines sous-populations leucocytaires, est d'une importance primordiale dans le diagnostic de certaines pathologies, tant en médecine humaine qu'en médecine vétérinaire.

L'hémoglobine est une chromo-protéine contenue dans les globules rouges (ou érythrocytes) du sang.

La mesure de la concentration de l'hémoglobine nécessite donc d'utiliser un réactif de lyse cellulaire capable de procéder à la lyse des érythrocytes ou globules rouges afin de libérer l'hémoglobine en vue de sa mesure.

Il est connu pour cela d'utiliser des réactifs contenant des ions cyanure capables de procéder à la lyse de l'érythrocyte et de transformer l'hémoglobine en un composé chromogène stable pour permettre sa détermination par mesure de colorimétrie. Un réactif de ce type est décrit en particulier dans le Brevet US 3 874 852. Ce réactif a pour inconvénient principal d'utiliser du cyanure. En outre, il ne permet pas d'identifier et de quantifier les sous-populations leucocytaires contenues dans l'échantillon de sang à analyser.

En effet, la détermination de sous-populations leucocytaires, en particulier des lymphocytes, des monocytes, des neutrophiles et des éosinophiles, s'avère d'une importance primordiale pour l'identification de certaines pathologies.

Différents réactifs, ne contenant pas de cyanure, ont déjà été proposés dans l'art antérieur pour la détermination des leucocytes dans un échantillon de sang.

Des exemples de tels réactifs sont décrits par exemple dans le document EP 0 325 710.

Si ces réactifs ont pour avantage de ne pas utiliser de cyanure, ils ont cependant pour inconvénient de ne pas permettre une identification et quantification précise des sous-populations leucocytaires du sang.

Ainsi, le réactif selon EP 0 325 710 permet par exemple de déterminer les éosinophiles présents dans un échantillon de sang, sans toutefois permettre d'identifier de façon précise chacune des autres sous-populations leucocytaires.

Par ailleurs, le document EP 0 424 871 décrit des réactifs permettant la différenciation de sous-populations leucocytaires. Mais ces réactifs éprouvent parfois des difficultés à vaincre les résitances membranaires qui sont présentes en pathologie humaine et en biologie vétérinaire.

L'invention a notamment pour but de surmonter les inconvénients précités.

Elle vise en particulier à procurer un réactif d'analyse hématologique pour la mesure de l'hémoglobine et la détermination des leucocytes dans un échantillon de sang qui permet notamment la lyse des érythrocytes ou globules rouges, ainsi que la mesure de l'hémoglobine, sans utilisation de composés cyanurés.

L'invention vise également à procurer un tel réactif qui permet de réaliser la quantification des leucocytes ou globules blancs totaux, l'identification d'au moins une sous-population leucocytaire, et la quantification d'au moins une sous-population leucocytaire.

Elle vise aussi à procurer un tel réactif qui peut être utilisé aussi bien en médecine humaine qu'en médecine vétérinaire.

Par ailleurs, l'invention vise à procurer un tel réactif qui est capable de surmonter les difficultées d'analyse liées aux résistances membranaires que l'on rencontre en pathologie humaine ainsi qu'en biologie vétérinaire.

L'invention propose à cet effet un réactif du type défini ci-dessus, lequel comprend :
- au moins un détergent de type cationique ;
- un composé de type glycoside, en particulier une saponine ;
- au moins un sel inorganique et/ou un agent osmotique et/ou leucoprotecteur ; et
- un tampon organique et/ou inorganique propre à ajuster sélectivement le pH du réactif, soit à une valeur sensiblement neutre (pH compris entre 5 et 8), soit à une valeur basique (pH compris entre 8 et 12).

Ainsi, le réactif hématologique de l'invention comprend au moins quatre composants principaux qui permettent d'atteindre les buts indiqués ci-dessus.

Le détergent de type cationique exerce une fonction de lyse des globules rouges ou érythrocytes, ce qui permet de libérer l'hémoglobine qui peut être ensuite déterminée par mesure d'absorbance.

Les détergents ioniques (anioniques et cationiques) sont principalement utilisés pour dissocier les complexes protéiques et solubiliser les protéines des membranes. Ils sont dits dénaturants.

En outre, ils ont une action rapide et non spécifique sur les populations cellulaires du sang (érythrocytes et leucocytes).

Le composé de type glycoside, en particulier une saponine, contribue à la lyse des globules rouges. En outre, il exerce une fonction de stabilisation à l'égard de l'hémoglobine, ce qui permet de la doser à une longueur d'ondes définie ; en particulier, ce composé contribue à détruire les membranes résistantes des globules rouges.

Les saponines, encore appelées saponosides, sont des glycosides ayant la capacité de lyser spécifiquement les érythrocytes, mais cette lyse est moins rapide que celle obtenue en présence des détergents ioniques. Lorsque les saponines sont utilisées en association avec d'autres détergents, elles permettent d'obtenir une lyse plus rapide, plus spécifique des érythrocytes, ce qui limite l'altération des leucocytes.

Par ailleurs, la structure chimique de ce type de composés, caractérisée par des squelettes carbonés à cycles multiples, permet la stabilisation physico-chimique des dérivés d'oxydation de l'hémoglobine.

Le sel inorganique intervient dans l'activité détergente. En outre, il est nécessaire à la mise en oeuvre d'une mesure par résistivité.

L'agent osmotique joue essentiellement un rôle leucoprotecteur, lorsque le réactif est utilisé à pH neutre, pour favoriser la différenciation des sous-populations leucocytaires.

Enfin, le tampon est un constituant clé car le pH du réactif est une caractéristique importante. En effet, suivant la valeur du pH, le réactif permet l'identification d'une ou plusieurs sous-population leucocytaire.

Lorsque le pH du réactif est ajusté à une valeur sensiblement neutre (pH compris entre 5 et 8), il est possible de quantifier et d'identifier les trois sous-populations leucocytaires suivantes : lymphocytes, monocytes et neutrophiles.

Lorsque le pH du réactif est ajusté à une valeur basique (pH compris entre 8 et 12), il permet en plus d'identifier et de quantifier les polynucléaires éosinophiles.

Ainsi, pour les applications nécessitant l'identification des polynucléaires éosinophiles, il faudra ajuster le pH du réactif à une valeur basique.

Par contre, si l'identification des éosinophiles n'est pas requise, le pH du réactif sera ajusté à une valeur sensiblement neutre.

Dans le réactif de l'invention, le détergent est avantageusement choisi parmi les composés suivants :
- les amines primaires, les acétates et chlorhydrates d'amines grasses ;
- les sels d'ammonium quaternaires et le bromure de triméthylcéthyl ammonium ;
- les amides de diamines substituées, cationisées par le sulfate d'éthyle, la diéthanolamino-propylamine, le diéthylamino-propylamide ; et
- les amides de diéthylènetriamine cyclisés.

Le détergent est avantageusement présent à une concentration comprise entre 0 et 50 g/l, et plus particulièrement d'environ 18 g/l.

Parmi les composés de type glycoside, on préfère tout particulièrement les saponines, encore appelées saponosides, qui peuvent être soit triterpéniques, soit stéroïdiques.

Dans le réactif de l'invention, le composé de type glycoside est avantageusement présent à une concentration comprise entre 0,5 et 20 g/l, et plus particulièrement d'environ 2g/l.

Le sel inorganique est avantageusement choisi parmi les composés suivants : chlorure, sulfate ou fluorure de sodium ou de potassium.

Ce sel inorganique est avantageusement présent à une concentration comprise entre 1 et 15 g/l, et plus particulièrement d'environ 8 g/l.

L'agent osmotique et/ou leucoprotecteur du réactif de l'invention est avantageusement choisi parmi le mannitol, le D-glucose et les composés analogues.

Cet agent osmotique et/ou leucoprotecteur est avantageusement présent à une concentration comprise entre 0 et 30 g/l, et plus particulièrement d'environ 2,5 g/l.

Le tampon organique et/ou inorganique est avantageusement choisi parmi les composés suivants :
- triéthanolamine ;
- phosphates hydrogénés de sodium et potassium ;
- acide N-[2-acétamido]-2 iminodiacétique ;
- acide N-[carbamoylméthyl] iminodiacétique ;
- 2 amino-2-méthyl 1,3 propanediol ;
- glycine ;
- carbonate de sodium ;
- acide citrique ; et
- tris (hydroxyméthyl) aminométhane.

Ce tampon est avantageusement présent à une concentration comprise entre 0 et 2% en poids.

Dans une première forme de réalisation de l'invention, le tampon ajuste le pH du réactif à une valeur comprise entre 5 et 8.

Dans une autre forme de réalisation, ce tampon ajuste le pH du réactif à une valeur comprise entre 8 et 12.

L'invention sera décrite maintenant en référence aux exemples non limitatifs suivants.

### EXEMPLE 1

On prépare un réactif à partir des composés ci-dessous, et dans les concentrations indiquées :

| Composés | Concentration |
|---|---|
| Chlorure de sodium | 9 g/l |
| C15 H34 NCl (détergent cationique) | 12 g/l |
| Saponine triterpénique | 2 g/l |
| Carbonate de sodium (tampon) | 2 g/l (0,2% en poids) |

Les composés sont mélangés et le pH est ajusté à une valeur basique comprise entre 8 et 12, à savoir 10,5.

A l'aide du réactif ainsi préparé on procède à deux analyses hématologiques en utilisant un automate d'hématologie commercialisé sous la marque ABX-Micros par la société française ABX.

Pour cela, on mélange au préalable un volume déterminé de l'échantillon de sang à analyser avec des volumes déterminés d'un diluant et du réactif ci-dessus.

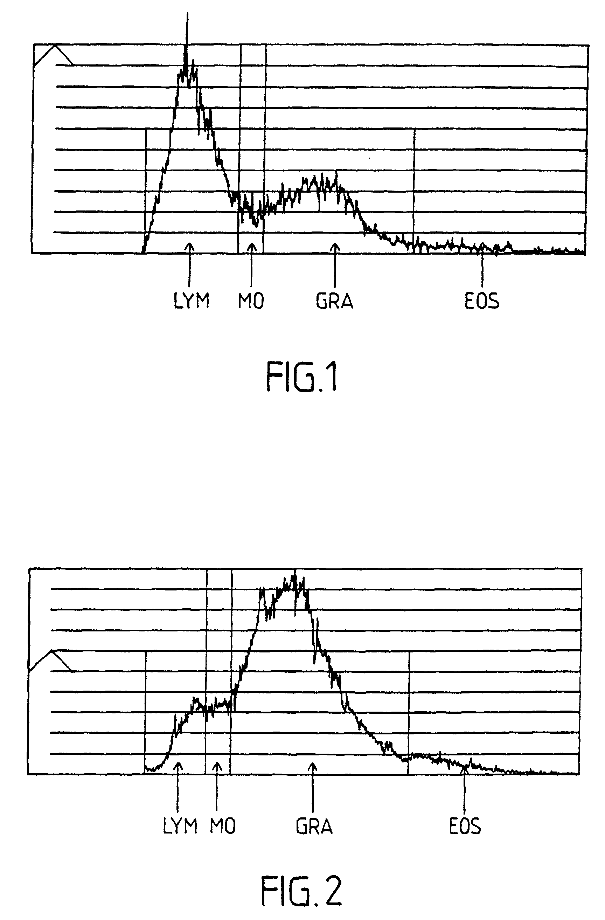

Dans l'exemple, on effectue des mesures sur du sang animal. Les résultats des mesures effectuées sur deux échantillons de sang différents sont représentés sur les figures 1 et 2.

Sur ces figures on a représenté le nombre de leucocytes en fonction d'une intensité relative. On constate l'existence de 4 sous-populations leucocytaires.

Il s'agit ici d'une analyse résistive si bien que l'on observe de droite à gauche, délimitées par des curseurs, les sous-populations suivantes : les lymphocytes (LYM), les monocytes (MO), les granulocytes (GRA) et les polynucléaires éosinophiles (EOS).

L'automate fournit les résultats de comptage suivants, qui indiquent, pour chaque sous-population leucocytaire, le nombre de cellules comptées et le pourcentage correspondant.

| ANALYSE 1 (figure 1) | | |
|---|---|---|
| LYM | 3956 cellules | 53,23% |
| MO | 420 cellules | 5,65% |
| GRA | 2576 cellules | 34,65% |
| EOS | 481 cellules | 6,47% |

| ANALYSE 2 (figure 2) | | |
|---|---|---|
| LYM | 1071 cellules | 9,34% |
| MO | 805 cellules | 7,00% |
| GRA | 8906 cellules | 77,52% |
| EOS | 706 cellules | 6,14% |

Ainsi le réactif du présent exemple permet d'identifier et de quantifier chacune des quatre sous-populations indiquées ci-dessus.

### EXEMPLE 2

On utilise le même réactif que dans l'exemple 1 et on procède à des analyses analogues sur deux échantillons différents de sang humain. Les mesures sont effectuées au moyen d'un automate hématologique ABX-Véga de la société ABX.

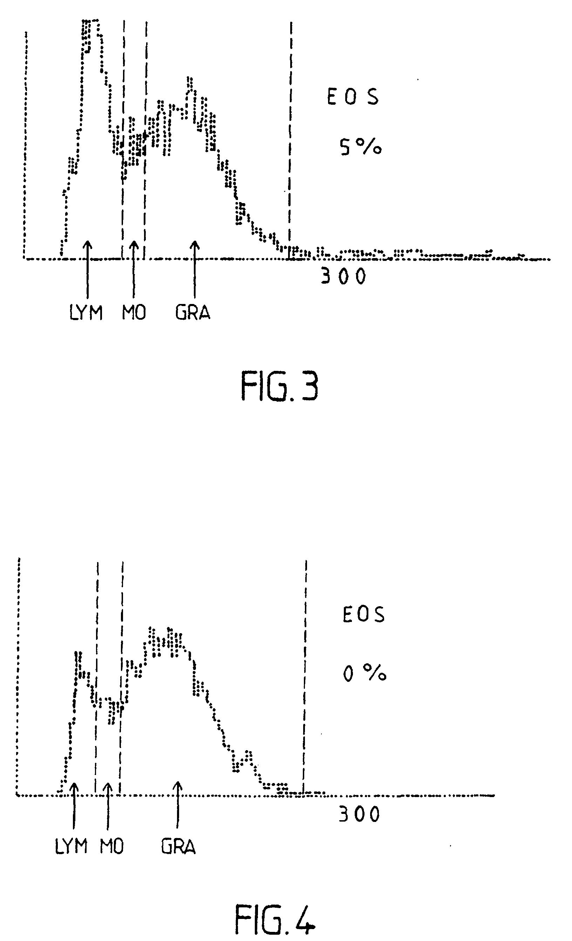

Les résultats sont représentés sur les figures 3 et 4.

Pour l'échantillon correspondant à la figure 3, l'analyse permet d'identifier et de quantifier les quatre sous-populations suivantes : les lymphocytes (LYM), les monocytes (MO), les granulocytes (GRA) et les polynucléaires éosinophiles (EOS). Ces derniers représentent 5% de la population totale.

Dans le cas de l'échantillon correspondant à la figure 4, l'analyse permet d'identifier et de quantifier les trois sous-populations suivantes : les lymphocytes (LYM), les monocytes (MO) et les granulocytes (GRA). En outre l'analyse révèle que l'échantillon ne contient pas de polynucléaires éosinophiles (taux de 0%).

### EXEMPLE 3

On utilise un réactif analogue à ceux des exemples 1 et 2, mais ajusté à un pH neutre, à savoir de 7,6%. On procède à des analyses analogues sur un échantillon de sang humain. Les mesures sont effectuées au moyen d'un automate hématologique ABX-Véga de la société ABX.

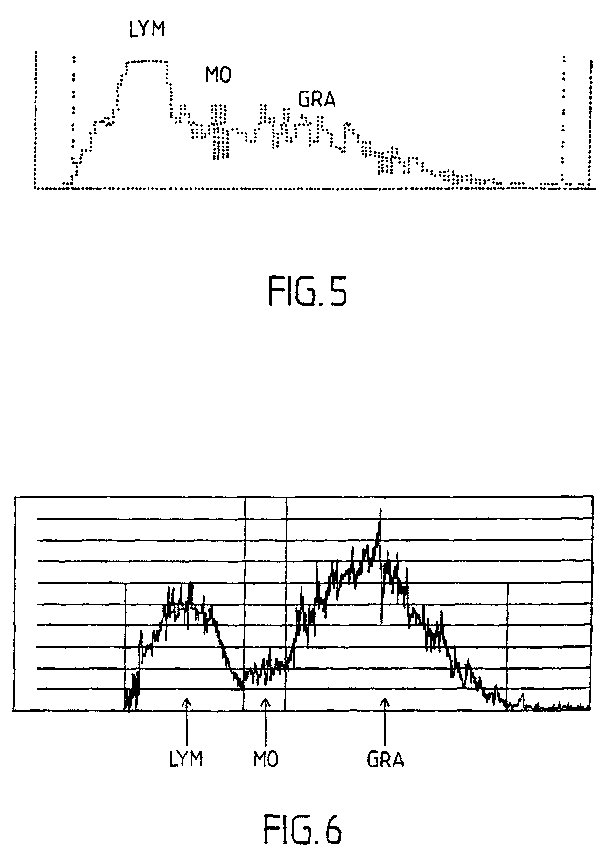

Les résultats des mesures sont représentés à la figure 5. Seules les trois principales sous-populations leucocytaires suivantes sont mises en évidence : les lymphocytes (LYM), les monocytes (MO) et les granulocytes (GRA).

### EXEMPLE 4

On utilise un réactif analogue à celui de l'exemple 3, donc ajusté à un pH neutre. On procède à des analyses analogues sur un échantillon de sang humain. Les mesures sont effectuées au moyen d'un automate hématologique du type ABX-Micros de la société ABX.

Les résultats des mesures sont représentés à la figure 6. Comme dans l'exemple 3, seules les trois principales sous-populations leucocytaires suivantes sont mises en évidence : les lymphocytes (LYM), les monocytes (MO) et les granulocytes (GRA).

## Revendications

1. Réactif pour la mesure de l'hémoglobine et la détermination des leucocytes dans un échantillon de sang,
**caractérisé en ce qu'**il comprend :
- au moins un détergent de type cationique ;
- un composé de type glycoside, en particulier une saponine;
- au moins un sel inorganique et/ou un agent osmotique et/ou leucoprotecteur ; et
- un tampon organique et/ou inorganique propre à ajuster sélectivement le pH du réactif, soit à une valeur sensiblement neutre (pH compris entre 5 et 8), soit à une valeur basique (pH compris entre 8 et 12).

2. Réactif selon la revendication 1, **caractérisé en ce que** le détergent est choisi parmi les composés suivants :
- les amines primaires, les acétates et chlorhydrates d'amines grasses ;
- les sels d'ammonium quaternaires et le bromure de triméthylcéthyl ammonium ;
- les amides de diamines substituées, cationisées par le sulfate d'éthyle, la diéthanolamino-propylamine, le diéthylamino-propylamide ; et
- les amides de diéthylènetriamine cyclisés.

3. Réactif selon l'une des revendications 1 et 2, **caractérisé en ce que** le détergent est présent à une concentration comprise entre 0 et 50 g/l.

4. Réactif selon l'une des revendications 1 à 3, **caractérisé en ce que** le composé de type glycoside est choisi parmi les saponines (ou saponosides).

5. Réactif selon l'une des revendications 1 à 4, **caractérisé en ce que** le composé de type glycoside est présent à une concentration comprise entre 0,5 et 20 g/l.

6. Réactif selon l'une des revendications 1 à 5, **caractérisé en ce que** le sel inorganique est choisi parmi les composés suivants : chlorure, sulfate ou fluorure de sodium ou de potassium.

7. Réactif selon l'une des revendications 1 à 6, **caractérisé en ce que** le sel inorganique est présent à une concentration comprise entre 1 et 15 g/l.

8. Réactif selon l'une des revendications 1 à 7, **caractérisé en ce que** l'agent osmotique et/ou leucoprotecteur est choisi parmi le mannitol, le D-glucose et analogues.

9. Réactif selon l'une des revendications 1 à 8, **caractérisé en ce que** l'agent osmotique et/ou leucoprotecteur est présent à une concentration comprise entre 0 et 30 g/l.

10. Réactif selon l'une des revendications 1 à 9, **caractérisé en ce que** le tampon organique et/ou inorganique est choisi parmi les composés suivants :
- triéthanolamine ;
- phosphates hydrogénés de sodium et potassium ;
- acide N-[2-acétamido]-2 iminodiacétique ;
- acide N-[carbamoylméthyl] iminodiacétique ;
- 2 amino-2-méthyl 1,3 propanediol ;
- glycine ;
- carbonate de sodium ;
- acide citrique ; et
- tris (hydroxyméthyl) aminométhane.

11. Réactif selon l'une des revendications 1 à 10, **caractérisé en ce que** le tampon organique et/ou inorganique est présent à une concentration comprise entre 0 et 2% en poids.

12. Réactif selon l'une des revendications 1 à 11, **caractérisé en ce que** le tampon ajuste le pH du réactif à une valeur comprise entre 5 et 8.

13. Réactif selon l'une des revendications 1 à 11, **caractérisé en ce que** le tampon ajuste le pH du réactif à une valeur comprise entre 8 et 12.

## Claims

1. Reagent for measuring the haemoglobin and determining the leukocytes in a blood sample, **characterized in that** it comprises:
- at least one detergent of the cationic type;
- a compound of the glycoside type, in particular a saponin;
- at least one inorganic salt and/or an osmotic and/or leukoprotective agent; and
- an organic and/or inorganic buffer which can selectively adjust the pH of the reagent, either to a substantially neutral value (pH between 5 and 8) or to a basic value (pH between 8 and 12).

2. Reagent according to Claim 1, **characterized in that** the detergent is chosen from the following compounds:
- primary amines, acetates and hydrochlorides of fatty amines;
- quaternary ammonium salts and trimethylcetylammonium bromide;
- amides of substituted diamines, cationized with ethyl sulphate, diethanolaminopropylamine, diethylaminopropylamide; and
- cyclized diethylenetriamine amides.

3. Reagent according to either of Claims 1 and 2, **characterized in that** the detergent is present at a concentration of between 0 and 50 g/l.

4. Reagent according to one of Claims 1 to 3, **characterized in that** the compound of the glycoside type is chosen from saponins (or saponosides).

5. Reagent according to one of Claims 1 to 4, **characterized in that** the compound of the glycoside type is present at a concentration of between 0.5 and 20 g/l.

6. Reagent according to one of Claims 1 to 5, **characterized in that** the inorganic salt is chosen from the following compounds: sodium chloride, sulphate or fluoride, or potassium chloride, sulphate or fluoride.

7. Reagent according to one of Claims 1 to 6, **characterized in that** the inorganic salt is present at a concentration of between 1 and 15 g/l.

8. Reagent according to one of Claims 1 to 7, **characterized in that** the osmotic and/or leukoprotective agent is chosen from mannitol, D-glucose and the like.

9. Reagent according to one of Claims 1 to 8, **characterized in that** the osmotic and/or leukoprotective agent is present at a concentration of between 0 and 30 g/l.

10. Reagent according to one of Claims 1 to 9, **characterized in that** the organic and/or inorganic buffer is chosen from the following compounds:
- triethanolamine;
- sodium hydrogen phosphate and potassium hydrogen phosphate;
- N-[2-acetamido]-2-iminodiacetic acid;
- N-[carbamoylmethyl]iminodiacetic acid;
- 2-amino-2-methyl-1,3-propanediol;
- glycine;
- sodium carbonate;
- citric acid; and
- tris(hydroxymethyl)aminomethane.

11. Reagent according to one of Claims 1 to 10, **characterized in that** the organic and/or inorganic buffer is present at a concentration of between 0 and 2% by weight.

12. Reagent according to one of Claims 1 to 11, **characterized in that** the buffer adjusts the pH of the reagent to a value of between 5 and 8.

13. Reagent according to one of Claims 1 to 11, **characterized in that** the buffer adjusts the pH of the reagent to a value of between 8 and 12.

## Patentansprüche

1. Reagens zur Messung des Hämoglobins und zur Bestimmung der Leukozyten in einer Blutprobe,
**dadurch gekennzeichnet, dass** es umfasst:
- mindestens ein Detergens des basischen Typs;
- mindestens eine Verbindung der Glycosidart, insbesondere ein Saponin;
- mindestens ein anorganisches Salz und/oder ein osmotisches Agens und/oder ein Leukoprotektoragens; und
- einen organischen und/oder anorganischen Puffer, der sich dazu eignet, den pH-Wert des Reagens selektiv entweder auf einen im wesentlichen neutralen Wert (pH zwischen 5 und 8) oder auf einen basischen Wert (pH zwischen 8 und 12) einzustellen.

2. Reagens nach Anspruch 1, **dadurch gekennzeichnet, dass** das Detergens aus den folgenden Verbindungen ausgewählt ist:
- primäre Amine, Acetate und Chlorhydrate von Fettaminen;
- quaternäre Ammoniumsalze und Trimethylcethylammoniumbromid;
- Amide von substituierten Diaminen, die durch Ethylsulfat kationisiert wurden, Dioethanolaminpropylamin, Diethylaminpropylamid; und
- Amide von zyklisiertem Diethylentriamin.

3. Reagens nach einem der Ansprüche 1 und 2, **dadurch gekennzeichnet, dass** das Detergens in einer Konzentration von 0 bis 50 g/l vorhanden ist.

4. Reagens nach einem der Ansprüche 1 bis 3, **dadurch gekennzeichnet, dass** die Verbindung der Glycosidart aus den Saponinen (oder Saponosiden) ausgewählt ist.

5. Reagens nach einem der Ansprüche 1 bis 4, **dadurch gekennzeichnet, dass** die Verbindung der Glycosidart in einer Konzentration von 0,5 bis 20 g/l vorhanden ist.

6. Reagens nach einem der Ansprüche 1 bis 5, **dadurch gekennzeichnet, dass** das anorganische Salz aus folgenden Verbindungen ausgewählt ist: Natriumchlorid, -sulfat oder -fluorid oder Kaliumchlorid, -sulfat oder -fluorid.

7. Reagens nach einem der Ansprüche 1 bis 6, **dadurch gekennzeichnet, dass** das anorganische Salz in einer Konzentration zwischen 1 und 15 g/l vorhanden ist.

8. Reagens nach einem der Ansprüche 1 bis 7, **dadurch gekennzeichnet, dass** das Osmotische Agens und/oder Leukoprotektoragens aus Manitol, D-Glukose und dergleichen ausgewählt ist.

9. Reagens nach einem der Ansprüche 1 bis 8, **dadurch gekennzeichnet, dass** das osmotische Agens und/oder Leukoprotektoragens in einer Konzentration von 0 bis 30 g/l vorhanden ist.

10. Reagens nach einem der Ansprüche 1 bis 9, **dadurch gekennzeichnet, dass** der organische und/oder anorganische Puffer aus den folgenden Verbindungen ausgewählt ist:
- Triethanolamin;
- hydrierten Natrium- und Kaliumphosphaten;
- N-[2-Acetamid]-2-Iminodiessigsäure;
- N-[Carbamoylmethyl]-Iminodiessigsäure;
- 2-Amino-2-Methyl-1,3-Propandiol;
- Glycin;
- Natriumcarbonat;
- Citronensäure; und
- Tris(hydroxymethyl)aminomethan.

11. Reagens nach einem der Ansprüche 1 bis 10, **dadurch gekennzeichnet, dass** dass der organische und/oder anorganische Puffer in einer Konzentration von 0 bis 2 Gew.-% vorhanden ist.

12. Reagens nach einem der Ansprüche 1 bis 11, **dadurch gekennzeichnet, dass** der Puffer den pH-Wert des Reagens auf 5 bis 8 einstellt.

13. Reagens nach einem der Ansprüche 1 bis 11, **dadurch gekennzeichnet, dass** der Puffer den pH-Wert des Reagens auf 8 bis 12 einstellt.
